# EUROPEAN PATENT SPECIFICATION

(11) **EP 3 375 412 B1**
(45) Date of publication and mention of the grant of the patent: **02.10.2019**
(21) Application number: 18164834.6
(22) Date of filing: 23.03.2016
(51) Int. Cl.: A61F 2/30, A61F 2/42

(54) **TALAR IMPLANT AND TOTAL JOINT REPLACEMENT SYSTEM COMPRISING SUCH A TALAR IMPLANT**
TALAR-IMPLANTAT UND TOTALGELENKERSATZ-SYSTEM MIT SOLCH EINEM TALAR-IMPLANTAT
IMPLANT ASTRAGALIEN ET SYSTÈME DE REMPLACEMENT TOTAL D'ARTICULATION COMPRENANT UN TEL IMPLANT ASTRAGALIEN

(30) Priority: 18.08.2015 US 201514828988
(43) Date of publication of application: 19.09.2018
(62) Divisional of application: 16161904.4
(73) Proprietor: Wright Medical Technology, Inc., Memphis, TN 38117 (US)
(72) Inventor: DHILLON, Braham K., Memphis, TN 38120 (US); MCGINLEY, Shawn E., Arlington, VA 38002 (US)
(74) Representative: Lavoix

(56) References cited:
- US-A1- 2005 125 070
- US-A1- 2005 288 792
- US-A1- 2009 082 875
- US-A1- 2012 010 718

## Description

The present invention relates to a talar implant. The invention also relates to a total joint replacement system.

### BACKGROUND

An ankle joint may become severely damaged and painful due to arthritis, prior ankle surgery, bone fracture, osteoarthritis, and/or one or more additional conditions. Options for treating the injured ankle have included anti-inflammatory and pain medications, braces, physical therapy, joint arthrodesis, and total ankle replacement.

Total ankle replacement generally comprises two components - tibial implant and a talar implant. The implants comprise articulation surfaces sized and configured to mimic the range of motion of the ankle joint. For example, the talar implant may comprise an implant sized and configured to mimic the talar dome and the tibial implant may comprise an articulation surface sized and configured to mimic articulation of the tibia. An articulating component may be located between the talar implant and the tibial implant.

Installation of a total ankle replacement can include forming one or more holes or cuts in a bone. For example, a hole may be drilled through the talus and into the tibia to create a channel for inserting a tibial stem. In some installations, additional bone is removed from the talus to make space for a talar stem extending from the talar portion. In order to interface the stem with the talar portion, the latter may define a cavity within which a head of the stem is received: in US 2009/082875, a spherical head frictionally engages a cylindrical wall of the cavity.

### SUMMARY

The present invention is a talar implant as defined in claim 1.

In various embodiments, an implant is disclosed. The implant comprises a body including a bone contact surface and an articulation surface located opposite the bone contact surface. The body defines a cavity extending from the bone contact surface into the body. A stem comprising a head and a longitudinal shaft is coupled at a predetermined angle to the head. The head is sized and configured to be received within the socket defined by the bone contact surface. The head is rotatable in at least one axis with respect to the body.

In various embodiments, a total joint replacement system is disclosed. The total joint replacement system comprises a tibial implant sized and configured to couple to a resected tibia and a talar implant sized and configured to couple to a resected talus. The talar implant includes a body including a bone contact surface and an articulation surface located opposite the bone contact surface. The body defines a cavity extending from the bone contact surface into the body. A stem comprising a head and a longitudinal shaft is coupled at a predetermined angle to the head. The head is sized and configured to be received within the socket defined by the bone contact surface. The head is rotatable in at least one axis within socket.

In various embodiments, an implant is disclosed. The implant includes a body including a bone contact surface and an articulation surface located opposite the bone contact surface. The body defines a socket extending from the bone contact surface into the body and a locking hole extending from the articulation surface to the socket. The locking hole is sized and configured to receive a locking fastener therein. A stem comprising a head and a longitudinal shaft is coupled at an angle to the head. The head defines a ball sized and configured to be received within the socket defined by the bone contact surface such that the longitudinal shaft is moveable in at least one axis with respect to the body.

### BRIEF DESCRIPTION OF THE FIGURES

The features and advantages of the present invention will be more fully disclosed in, or rendered obvious by the following detailed description of the preferred embodiments, which are to be considered together with the accompanying drawings wherein like numbers refer to like parts and further wherein:
FIG. 1 illustrates an anatomic view of an ankle joint.
FIG. 2 illustrates an ankle joint having a total ankle replacement system therein.
FIG. 3 illustrates one example of an anchoring stem having a head configured to rotatably couple to an implant.
FIG. 4 illustrates an implant including a cavity sized and configured to receive the head of the stem of FIG. 3 therein.
FIG. 5 illustrates the stem of FIG. 3 secured to the implant of FIG. 4 by a set screw.
FIG. 6 illustrates various positional relationships between the stem of FIG. 3 and the implant of FIG. 4.
FIGS. 7A-7C illustrate various embodiments of stems having anti-movement features formed thereon.

### DETAILED DESCRIPTION

The description of the exemplary embodiments is intended to be read in connection with the accompanying drawings, which are to be considered part of the entire written description. In the description, relative terms such as "lower," "upper," "horizontal," "vertical," "proximal," "distal," "above," "below," "up," "down," "top" and "bottom," as well as derivatives thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

In various embodiments, the present disclosure generally provides a talar implant for use with a total ankle replacement system. The talar implant includes a cutout or cavity formed in a bone contact side of the implant. The cavity is sized and configured to receive a head of an anchoring stem therein. A longitudinal shaft of the stem extends from the head at an angle. The cavity and the head are rotatably coupled such that the longitudinal shaft of the stem can be rotated to at any desired angle within a predetermined range of angles relative to a central axis of the implant.

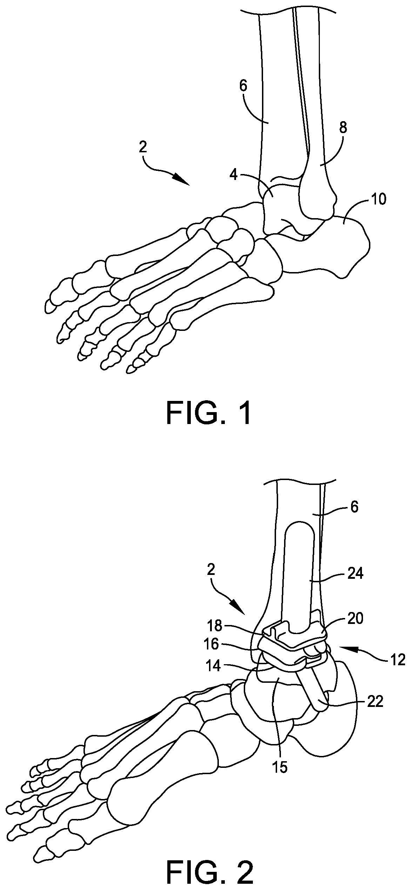

FIG. 1 illustrates an anatomic view of an ankle joint 2. The ankle joint 2 comprises a talus 4 in contact with a tibia 6 and a fibula 8. A calcaneus 10 is located adjacent to the talus 4. In total ankle replacements, the talus 4 and the tibia 6 may be resected, or cut, to allow insertion of a talar implant and a tibial implant. FIG. 2 illustrates the ankle joint 2 of FIG. 1 having a total ankle replacement system 12 inserted therein.

The total ankle replacement system 12 comprises a talar implant 14 and a tibial implant 18. The talar implant 14 comprises a body defining a talar articulation surface 16 (or talar dome). A stem 22 extends into the talus 4 to anchor the talar implant 14 to the talus 4. The tibial implant 18 is sized and configured for installation into the tibia 6. The tibial implant 18 comprises a body having an articulation surface 20 and a tibial stem 24 extending into the tibia 6 to anchor the tibial implant 18. The talar joint surface 16 and the tibial joint surface 20 are mutually sized and configured to articulate. The joint surfaces 16, 20 replace the natural ankle joint surfaces, which are removed, to restore a range of motion that mimics the natural joint. One or more holes may be formed in the tibia and/or the talus prior to and during insertion of the tibial implant 18 or the talar implant 12. For example, in some embodiments, a hole is drilled starting in the bottom of the talus, extending through the talus and into the tibia. The hole may comprise, for example, a 6mm hole configured to receive the stem 24 of the tibial implant 18.

The joint surfaces 16, 20 may be made of various materials, such as, for example, polyethylene, high molecular weight polyethylene (HMWPE), rubber, titanium, titanium alloys, chrome cobalt, surgical steel, and/or any other suitable metal, ceramic, sintered glass, artificial bone, and/or any combination thereof. The joint surfaces 16, 20 may comprise different materials. For example, the tibial joint surface 20 may comprise a plastic or other non-metallic material and the talar joint surface 16 may comprise a metal surface. Those skilled in the art will recognize that any suitable combination of materials may be used.

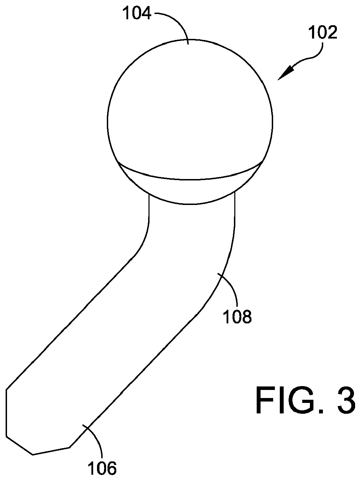

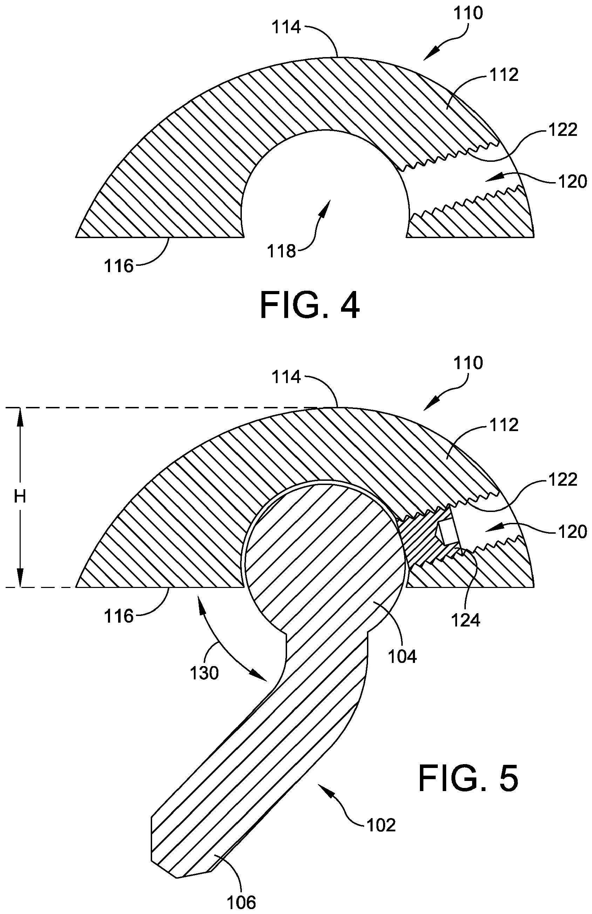

An implant, such as, for example, a talar implant 14, may be coupled to a talus by a stem. FIG. 3 illustrates one embodiment of an anchoring stem 102. The stem 102 comprises a head 104 and a longitudinal shaft 106. The longitudinal shaft 106 is coupled to the head 104 by a curved section 108. The curved section 108 maintains the longitudinal shaft 106 at a fixed angle with respect to the head 104. The stem 102 is configured to interface with an implant, such as, for example, the implant 110 illustrated in FIG. 4, such that the longitudinal shaft 106 of the stem 102 is rotatable with respect to the implant 102. For example, in the illustrated embodiment, the head 104 comprises a ball-type head configured to rotate within a socket defined by an implant 110.

FIG. 4 illustrates a cross-sectional view of an implant 110 defining a cavity 118 therein for receiving the head 104 of the anchoring stem 102. The implant 110 comprises a body 112 having an articulation surface 114 and a bone contact surface 116 opposite the articulation surface 114. A cavity 118 is formed in the body 112 and extends from the bone contact surface 116 a predetermined distance into the body 112. The predetermined distance is less than the distance between the articulation surface 114 and the bone contact surface 116. The cavity 118 is sized and configured to receive a head 104 of a stem 102 therein such that the stem 102 is rotatable within the cavity 118 to adjust a position of the longitudinal shaft 106 of the stem 102 with respect to a central axis of the implant 102.

In some embodiments, a locking hole 120 is formed in the implant 110. The locking hole 120 extends through the body 112 from the articulation surface 114 into the cavity 118. The locking hole 120 is sized and configured to receive a locking fastener 124, such as, for example, a set screw and/or other locking device therein. In some embodiments, the locking hole 120 comprises a plurality of internal threads 122 sized and configured to couple to the locking fastener 124 (see FIG. 5). The locking fastener 124 is inserted into the locking hole 120 to lock the steam 102 at a selected angle. In some embodiments, the locking fastener 124 is sized and configured to be flush with the articulation service 114 in a locked position, such that the articulation surface 114 is continuous over the locking hole 120 when the locking fastener 124 is in a locked position. In other embodiments, a cap (not shown) is inserted into a proximal end of the locking hole 120 to provide a smooth, continuous surface on the articulation surface 114 and prevent potential rubbing or other issues caused by the locking hole 120.

FIG. 5 illustrates a cross-sectional view of the stem 102 of FIG. 3 and the implant 110 of FIG. 4 mated together. As shown in FIG. 5, the head 104 of the stem 102 is sized and configured to fit within the cavity 118 defined by the body 112 of the implant 110. In some embodiments, the cavity 118 has a depth greater than half the diameter of the head 104 but less than the total diameter of the head 104. In other embodiments, the cavity 118 may have a greater or lesser depth, such as, for example, a depth equal to the diameter of the head 104, a depth greater than the diameter of the head 104, and/or any other suitable depth. The stem 102 is rotatable within the cavity 118 to allow the longitudinal shaft 106 of the stem 102 to be positioned at a selected angle with respect to a central axis of the implant 110. In some embodiments, after the head 104 is inserted into the cavity 118, a locking fastener 124 is driven into the locking hole 120 and into contact with the plurality of threads 122. The locking fastener 124 is driven to a distal position within the locking hole 120. The locking fastener 124 maintains the ball-style head 104 in a fixed position with the talar dome 110. In some embodiments, the locking fastener 124 is configured to allow movement in a first direction, such as, for example, rotation of the head 104 while preventing movement in a second direction, such as, for example, lateral movement of the head 104.

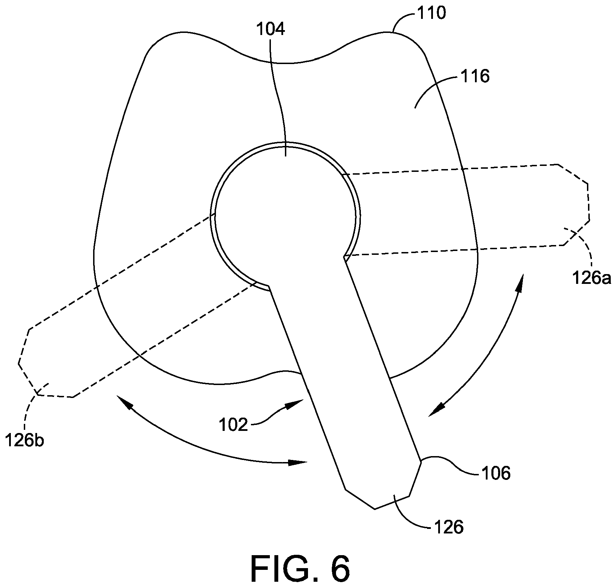

In the illustrated example, the ball-and-socket connection between the ball-type head 104 of the stem 102 and the cavity 118 of the talar dome 110 allows the stem 102 to be positioned at any selected angle. FIG. 6 illustrates various positions of the stem 102 with respect to the talar dome 110. As shown in FIG. 6, the stem 102 may be rotated about a center point of defined by the head 104. For example, the stem 102 is shown with the longitudinal shaft 106 in an initial position 126. The stem 102 may be rotated to position the longitudinal shaft 106 at various angles with respect to a central axis of the implant 110. For example, two additional positions 126a, 126b are illustrated in phantom in FIG. 6. In some embodiments, the stem 102 may be continuously rotated 360°. In other embodiments, the stem 102 may have a range of rotation less than 360°. In some embodiments, the stem 102 is positioned at a specific angle of rotation and locked into place, for example, by a locking fastener 124. In other embodiments, the stem 102 is locked into a specific angle of rotation when inserted into a hole formed in a bone, such as, for example, a talus.

In some embodiments, the head 104 and the cavity 118 enable the stem 102 to be angled to adjust the spacing between the longitudinal shaft 106 and the bone contact surface 114 of the implant 110. For example, as shown in FIG. 4, the longitudinal shaft 106 comprises an angle 130 with respect to the bone contact surface 114. In some embodiments, the angle 130 is adjustable. After the angle 130 has been set, the locking fastener 124 may be tightened to maintain the stem 102 at a fixed angle and/or a fixed rotation. In some embodiments, the angle 130 is determined when the implant 110 is coupled to a bone, such as, for example, a talus.

The longitudinal stem 106 is configured to prevent the stem 102 from pulling out of a hole formed in a bone. For example, the longitudinal stem 106 can include one or more features that couple the stem to an internal wall of the hole to prevent the longitudinal stem 106 from moving within the hole. According to the invention, the longitudinal stem 106 includes a grooved stem optionally combined with one or more splines and/or a friction coating and/or any other suitable feature to prevent the stem 102 from pulling out of the hole. In some embodiments, the longitudinal stem 106 extends a distance equal to or less than the distance H illustrated in FIG. 4, to provide for easy insertion of the longitudinal stem 106 into the bone. In some embodiments, the longitudinal stem 106 extends a distance greater than the distance H.

In various embodiments, the head 104 of the stem 102 and the cavity 118 provide multi-directional freedom of movement to the longitudinal shaft 106 with respect to the bone contact surface 116 of the implant 110. For example, in the illustrated embodiment, the ball-and-socket connection between the head 104 and the implant 110 provides free rotational and angular movement to the longitudinal shaft 106. The longitudinal shaft 106 can be positioned at any advantageous angle and/or rotational position with respect to the implant 110. The freedom of movement provided by the ball-and-socket connection allows the implant 110 to be positioned by the surgeon at any angle/rotation during implantation and anchoring of the implant 110. The freedom of movement provided by the ball-and-socket connection further allows for compensation of variances in the angles/positions of holes made in a bone during implantation without the need to re-ream and/or drill additional holes.

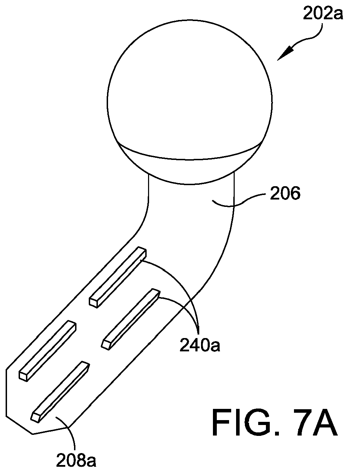

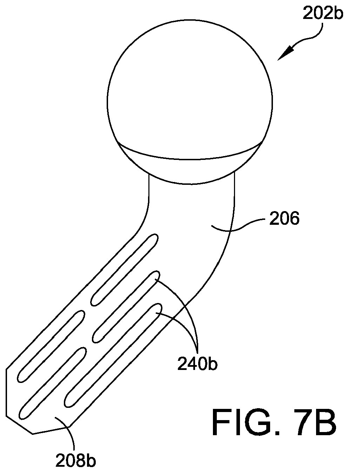

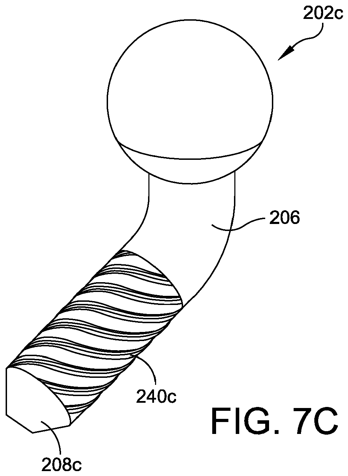

FIGS. 7A-7C illustrate longitudinal shafts 206a-206c having anti-movement features 240 formed thereon. As shown in FIG. 7A, the anti-movement features 240 are formed on at least a portion of the longitudinal shaft 206a. The anti-movement features 240 provide resistance, such as, for example, frictional resistance, to backward movement of the longitudinal shaft 206a within a hole formed in a bone. The anti-movement features 240 may comprise splines (see FIG. 7A), grooves (see FIG. 7B), a friction coating (see FIG. 7C), and/or any other suitable anti-movement feature 240. Although the anti-movement features 240 are illustrates as being disposed over the entire circumference of the longitudinal shaft 206a-206c, it will be appreciated that the anti-movement features 240 may be limited to a specific portion of the longitudinal shaft 206a-206c, such as, for example, a distal end and/or a proximal end.

In various embodiments, an implant is disclosed. The implant comprises a body including a bone contact surface and an articulation surface located opposite the bone contact surface. The body defines a cavity extending from the bone contact surface into the body. A stem comprising a head and a longitudinal shaft is coupled at a predetermined angle to the head. The head is sized and configured to be received within the socket defined by the bone contact surface. The head is rotatable in at least one axis with respect to the body.

In some embodiments, the stem is rotatable about a central axis of the head of the stem. In some embodiments, the stem is rotatable such that an angle between the longitudinal shaft and the bone contact surface of the body is adjustable. The body can define a locking hole extending from the articulation surface to the cavity. The locking hole is sized and configured to receive a locking fastener therein. The locking fastener is configured to prevent movement of the stem in at least one direction.

In some embodiments, the cavity comprises a socket. The head of the stem comprises a ball sized and configured to be received within the socket. In some embodiments, the stem comprises one or more anti-movement features configured to prevent movement of the stem with respect to the bone. The anti-movement features can comprise splines, grooves, and/or a friction coating formed on the stem.

In some embodiments, the stem extends a first distance. The distance between the bone contact surface and the articulation surface comprises a second distance. The first distance is less than the second distance.

In various embodiments, a total joint replacement system is disclosed. The total joint replacement system comprises a tibial implant sized and configured to couple to a resected tibia and a talar implant sized and configured to couple to a resected talus. The talar implant includes a body including a bone contact surface and an articulation surface located opposite the bone contact surface. The body defines a cavity extending from the bone contact surface into the body. A stem comprising a head and a longitudinal shaft is coupled at a predetermined angle to the head. The head is sized and configured to be received within the socket defined by the bone contact surface. The head is rotatable in at least one axis within socket.

In some embodiments, the body defines a locking hole extending from the articulation surface to the cavity. The locking hole is sized and configured to receive a locking fastener therein. The locking fastener is configured to prevent movement of the stem in at least one direction.

In some embodiments, the cavity comprises a socket and the head of the stem comprises a ball sized and configured to be received within the socket. The stem comprises one or more anti-movement features to prevent movement of the stem with respect to the bone. The one or more anti-movement features can comprise at least one of a spline, a groove, and/or a friction coating formed on the stem.

In various embodiments, an implant is disclosed. The implant includes a body including a bone contact surface and an articulation surface located opposite the bone contact surface. The body defines a socket extending from the bone contact surface into the body and a locking hole extending from the articulation surface to the socket. The locking hole is sized and configured to receive a locking fastener therein. A stem comprising a head and a longitudinal shaft is coupled at an angle to the head. The head defines a ball sized and configured to be received within the socket defined by the bone contact surface such that the longitudinal shaft is moveable in at least one axis with respect to the body. In some embodiments, the stem comprises one or more anti-movement features to prevent movement of the stem with respect to the bone.

Although the subject matter has been described in terms of exemplary embodiments, it is not limited thereto. Rather, the appended claims should be construed broadly, to include other variants and embodiments, which may be made by those skilled in the art.

## Claims

1. A talar implant comprising:
a talar dome (110) including a bone contact surface (116) and an articulation surface (114) located opposite the bone contact surface, the talar dome defining a cavity (118) extending from the bone contact surface into the talar dome; and
a stem (102; 202a; 202b; 202c) comprising a head (104) and a longitudinal shaft (106; 206) coupled at a predetermined angle to the head,
wherein the head (104) is sized and configured to be received within the cavity (118),
wherein the head (104) is rotatable about at least one axis with respect to the talar dome (110), and
wherein the longitudinal shaft (106; 206) comprises one or more features (240) configured to interface with a wall of a hole formed in a bone to prevent movement of the stem with respect to the bone,
**characterized in that** the one or more features (240) comprises one or more grooves (240b) formed on the longitudinal shaft (106; 206).

2. The implant of claim 1, wherein the one or more features (240) comprises one or more splines (240a) formed on the longitudinal shaft (106; 206).

3. The implant of any one of claims 1 to 2, wherein the one or more features (240) comprises a friction coating formed on the longitudinal shaft (106; 206).

4. The implant of any one of claims 1 to 3, wherein the stem (102; 202a; 202b; 202c) is rotatable about a central axis of the head (104) of the stem.

5. The implant of any one of claims 1 or 4, wherein the stem (102; 202a; 202b; 202c) is rotatable such that an angle between the longitudinal shaft (106; 206) and the bone contact surface (116) of the talar dome (110) is adjustable.

6. The implant of any one of claims 1 to 5, wherein the cavity (118) comprises a socket, and wherein the head (104) of the stem (102; 202a; 202b; 202c) comprises a ball sized and configured to be received within the socket.

7. The implant of any one of claims 1 to 6, wherein the stem (102; 202a; 202b; 202c) extends a first distance, wherein the distance between the bone contact surface (116) and the articulation surface (114) comprises a second distance, and wherein the first distance is less than the second distance.

8. The implant of any one of claims 1 to 7, wherein the longitudinal shaft (106; 206) is coupled to the head (104) by a curved section (108) of the stem (102; 202a; 202b; 202c).

9. The implant of any one of claims 1 to 8, wherein the cavity (118) has a depth and the head (104) has a diameter, and wherein the depth is greater than half the diameter but less than the diameter.

10. The implant of claim 9, wherein the talar dome (110) defines a locking hole (120) extending from the articulation surface (114) to the cavity (118) such that the locking hole (120) is configured to receive a locking fastener (124) such that the locking fastener (124) contacts the head (104) of the stem (102; 202a; 202b; 202c).

11. A total joint replacement system, comprising:
a tibial implant (18) sized and configured to couple to a resected tibia (6); and
a talar implant (14) sized and configured to couple to a resected talus (4), the talar implant being according to any one of the claims 1 to 10.

## Patentansprüche

1. Talar-Implantat, umfassend:
einen Talusdom (110), darin eingeschlossen eine Knochenkontaktfläche (116) und eine Gelenkfläche (114), die sich gegenüber der Knochenkontaktfläche befindet, wobei der Talusdom einen Hohlraum (118) definiert, der sich von der Knochenkontaktfläche in den Talusdom erstreckt; und
einen Stamm (102; 202a; 202b; 202c), der einen Kopf (104) und einen länglichen Schaft (106; 206) umfasst, der in einem vorbestimmten Winkel an den Kopf gekoppelt ist,
wobei der Kopf (104) abgemessen und konfiguriert ist, um innerhalb des Hohlraums (118) aufgenommen zu werden,
und wobei der Kopf (104) um mindestens eine Achse mit Bezug auf den Talusdom (110) drehbar ist; und
wobei der längliche Schaft (106; 206) ein oder mehrere Merkmale (240) umfasst, die konfiguriert sind, um mit einer Wand eines Lochs eine Schnittstelle zu bilden, das gebildet ist, um die Bewegung des Stamms mit Bezug auf den Knochen zu verhindern,
**dadurch gekennzeichnet, dass** eines oder mehrere Merkmale (240) eine oder mehrere Nuten (240b) umfasst, die auf dem länglichen Schaft (106; 206) gebildet sind.

2. Implantat nach Anspruch 1, wobei das eine oder die mehreren Merkmale (240) eine oder mehrere Nuten (240a) umfasst, die auf dem länglichen Schaft (106; 206) gebildet sind.

3. Implantat nach einem der Ansprüche 1 bis 2, wobei die eine oder die mehreren Nuten (240) eine Reibungsbeschichtung umfassen, die auf dem länglichen Schaft (106; 206) gebildet ist.

4. Implantat nach einem der Ansprüche 1 bis 3, wobei der Stamm (102; 202a; 202b; 202c) um eine zentrale Achse des Kopfes (104) des Stamms drehbar ist.

5. Implantat nach einem der Ansprüche 1 bis 4, wobei der Stamm (102; 202a; 202b; 202c) derart drehbar ist, dass ein Winkel zwischen dem länglichen Schaft (106; 206) und der Knochenkontaktfläche (116) des Talusdoms (110) einstellbar ist.

6. Implantat nach einem der Ansprüche 1 bis 5, wobei der Hohlraum (118) einen Sockel umfasst, und wobei der Kopf (104) des Stamms (102; 202a; 202b; 202c) eine Kugel umfasst, die abgemessen und konfiguriert ist, um innerhalb des Sockels aufgenommen zu werden.

7. Implantat nach einem der Ansprüche 1 bis 6, wobei sich der Stamm (102; 202a; 202b; 202c) über eine erste Distanz erstreckt, wobei die Distanz zwischen der Knochenkontaktfläche (116) und der Gelenkfläche (114) eine zweite Distanz umfasst, und wobei die erste Distanz kleiner als die zweite Distanz ist.

8. Implantat nach einem der Ansprüche 1 bis 7, wobei der längliche Schaft (106, 206) mit dem Kopf (104) durch einen gekrümmten Abschnitt (108) des Stamms (102; 202a; 202b; 202c) gekoppelt ist.

9. Implantat nach einem der Ansprüche 1 bis 8, wobei der Hohlraum (118) eine Tiefe und der Kopf (104) einen Durchmesser aufweist, und wobei die Tiefe größer als der halbe Durchmesser ist jedoch kleiner als der Durchmesser.

10. Implantat nach Anspruch 9, wobei der Talusdom (110) ein Verriegelungsloch (120) definiert, das sich von der Gelenkfläche (114) zum Hohlraum (118) erstreckt, so dass das Verriegelungsloch (120) konfiguriert ist, um eine Verriegelungsbefestigung (124) aufzunehmen, so dass die Verriegelungsbefestigung (124) mit dem Kopf (104) des Stamms (102; 202a; 202b; 202c) in Kontakt kommt.

11. Totalgelenkersatz-System, umfassend,
ein Schienbeinimplantat (18), das abgemessen und konfiguriert ist, um an ein reseziertes Schienbein (6) gekoppelt zu sein; und
ein Talar-Implantat (14), das abgemessen und konfiguriert ist, um an einen resezierten Talus (4) gekoppelt zu sein, wobei das Talar-Implantat gemäß einem der Ansprüche 1 bis 10 ist.

## Revendications

1. Implant astragalien comprenant :
un dôme astragalien (110) comprenant une surface de contact d'os (116) et une surface d'articulation (114) positionnée à l'opposé de la surface de contact d'os, le dôme astragalien définissant une cavité (118) s'étendant à partir de la surface de contact d'os dans le dôme astragalien ; et
une tige (102 ; 202a ; 202b ; 202c) comprenant une tête (104) et un arbre longitudinal (106 ; 206) couplé, selon un angle prédéterminé, à la tête,
dans lequel la tête (104) est dimensionnée et configurée pour être reçue à l'intérieur de la cavité (118),
dans lequel la tête (104) peut tourner autour d'au moins un axe par rapport au dôme astragalien (110), et
dans lequel l'arbre longitudinal (106 ; 206) comprend une ou plusieurs caractéristiques (240) configurées pour s'interfacer avec une paroi d'un trou formé dans un os pour empêcher le mouvement de la tige par rapport à l'os,
**caractérisé en ce que** les une ou plusieurs caractéristiques (240) comprennent une ou plusieurs rainures (240b) formées sur l'arbre longitudinal (106 ; 206).

2. Implant selon la revendication 1, dans lequel les une ou plusieurs caractéristiques (240) comprennent une ou plusieurs cannelures (240a) formées sur l'arbre longitudinal (106 ; 206).

3. Implant selon l'une quelconque des revendications 1 à 2, dans lequel les une ou plusieurs caractéristiques (240) comprennent un revêtement de friction formé sur l'arbre longitudinal (106 ; 206).

4. Implant selon l'une quelconque des revendications 1 à 3, dans lequel la tige (102 ; 202a ; 202b ; 202c) peut tourner autour d'un axe central de la tête (104) de la tige.

5. Implant selon l'une quelconque des revendications 1 ou 4, dans lequel la tige (102 ; 202a ; 202b ; 202c) peut tourner de sorte qu'un angle entre l'arbre longitudinal (106 ; 206) et la surface de contact d'os (116) du dôme astragalien (110) est ajustable.

6. Implant selon l'une quelconque des revendications 1 à 5, dans lequel la cavité (118) comprend une douille, et dans lequel la tête (104) de la tige (102 ; 202a ; 202b ; 202c) comprend une bille dimensionnée et configurée pour être reçue à l'intérieur de la douille.

7. Implant selon l'une quelconque des revendications 1 à 6, dans lequel la tige (102 ; 202a ; 202b ; 202c) s'étend sur une première distance, dans lequel la distance entre la surface de contact d'os (116) et la surface d'articulation (114) comprend une seconde distance, et dans lequel la première distance est inférieure à la seconde distance.

8. Implant selon l'une quelconque des revendications 1 à 7, dans lequel l'arbre longitudinal (106 ; 206) est couplé à la tête (104) par une section incurvée (106) de la tige (102 ; 202a ; 202b ; 202c).

9. Implant selon l'une quelconque des revendications 1 à 8, dans lequel la cavité (118) a une profondeur et la tête (104) a un diamètre, et dans lequel la profondeur est inférieure à la moitié du diamètre mais inférieure au diamètre.

10. Implant selon la revendication 9, dans lequel le dôme astragalien (110) définit un trou de verrouillage (120) s'étendant à partir de la surface d'articulation (114) jusqu'à la cavité (118), de sorte que le trou de verrouillage (120) est configuré pour recevoir une fixation de verrouillage (124) de sorte que la fixation de verrouillage (124) est en contact avec la tête (104) de la tige (102 ; 202a ; 202b ; 202c).

11. Système de remplacement total d'articulation comprenant :
un implant tibial (18) dimensionné et configuré pour se coupler à un tibia réséqué (6) ; et
un implant astragalien (14) dimensionné et configuré pour se coupler à un astragale réséqué (4), l'implant astragalien étant selon l'une quelconque des revendications 1 à 10.
